# NOUVEAU FASCICULE DE BREVET EUROPEEN

(11) **EP 0 403 401 B2**
(45) Date de publication et mention de la décision concernant l'opposition: **03.11.1999**
(45) Mention de la délivrance du brevet: 16.03.1994
(21) Numéro de dépôt: 90420241.3
(22) Date de dépôt: 18.05.1990
(51) Int. Cl.: A61M 1/16, G01F 25/00

(54) **Méthode de contrôle de l'ultrafiltration et dispositif pour sa mise en oeuvre**
Verfahren zur Kontrolle der Ultrafiltration und Vorrichtung zur Ausführung des Verfahrens
Method to control ultrafiltration and device for carrying out this method

(30) Priorité: 29.05.1989 FR 8907275
(43) Date de publication de la demande: 19.12.1990
(73) Titulaire: HOSPAL INDUSTRIE, F-69883 Meyzieu Cédex (FR)
(72) Inventeur: Chevallet, Jacques, F-69360 Serezin du Rhône (FR)
(74) Mandataire: Lejeune, Daniel

(56) Documents cités:
- EP-A- 0 213 050
- EP-A- 0 266 590
- DE-A- 384 183
- US-A- 3 939 069
- US-A- 4 334 988
- US-A- 4 831 866

## Description

La présente invention concerne le domaine technique du traitement extracorporel du sang par un rein artificiel. Plus particulièrement, la présente invention concerne le contrôle de la quantité de liquide extraite du sang par ultrafiltration, grâce à un contrôle du pompage du liquide extrait du circuit de liquide de dialyse.

En effet, lors du traitement du sang d'un patient souffrant d'insuffisance rénale, il faut éliminer du sang, d'une part les impuretés telles que l'urée et d'autre part, l'excès d'eau qui s'est accumulé entre deux séances. Ce traitement du sang se fait dans un échangeur grâce à une membrane permettant à la fois la dialyse et l'ultrafiltration. Le sang à traiter circule d'un côté de la membrane alors que le liquide de traitement appelé liquide de dialyse circule de l'autre côté de la membrane. Lorsqu'une différence de pression existe entre les deux compartiments de l'échangeur, du liquide peut traverser la membrane par convexion, afin de tendre à rétablir l'équilibre des pressions. Ainsi, en créant une dépression dans le compartiment parcouru par le liquide de dialyse, une fraction de la partie liquide du sang traverse la membrane pour se retrouver dans le liquide de dialyse. Il est alors nécessaire, dans ce type d'opération, de contrôler le plus précisément possible, les pressions s'exerçant dans chacun des compartiments afin de contrôler, par voie de conséquence, l'ultrafiltration du sang.

Parmi les moyens de contrôle possibles, ceux décrits dans le brevet US 3.939.069 relatifs au contrôle volumétrique de l'ultrafiltration sont parmi les plus fiables. Selon cette technique, lorsque le circuit de liquide de dialyse est un circuit indéformable et fermé du point de vue des pressions, toute quantité de liquide extraite du circuit provoque une dépression dans le circuit. Cette dépression se traduit, au niveau de l'échangeur, par un passage de liquide du sang vers le liquide de dialyse jusqu'au rétablissement de l'équilibre des pressions, c'est-à-dire jusqu'à ce que la quantité de liquide ultrafiltré du sang soit égale à la quantité de liquide extraite du circuit. Ainsi, le pompage d'une quantité de liquide provenant d'un circuit fermé de liquide de dialyse permet d'imposer l'ultrafiltration du sang. On assimilera donc dans la suite du présent texte, l'extraction de liquide du circuit de liquide de dialyse en vue de provoquer l'ultrafiltration du sang, à l'ultrafiltration du sang elle-même.

Le problème à résoudre par la présente invention concerne alors le contrôle du pompage. En effet, si l'on veut être précis dans le contrôle de l'ultrafiltration du sang, il faut tout d'abord être précis dans le contrôle du pompage du liquide extrait du circuit de liquide de dialyse. Cette exigence est due au fait que ce contrôle de l'ultrafiltration présente pour la sécurité et le bien-être du patient, une très grande importance dans le traitement du sang par un rein artificiel car, dans le cas où l'ultrafiltration du sang n'est pas correctement effectuée, le patient peut connaître des troubles importants tels que nausées, maux de tête, crampes, etc... connus sous le terme de syndrôme de déséquilibre.

Le pompage de l'ultrafiltration du sang peut être effectué grâce à une pompe dont le fonctionnement se traduit par l'émission d'impulsions correspondant chacune au passage d'une certaine quantité de liquide. La correspondance entre les impulsions émises et la quantité de liquide pompé est une caractéristique donnée généralement par le fournisseur de pompes. Cependant, cette caractéristique peut varier en fonction de l'usure de la pompe et en fonction de ses conditions d'utilisation. Ainsi, par exemple, la pression s'exerçant en amont de la pompe exerce une grande influence sur les performances de la pompe. Or, dans le cas du pompage de l'ultrafiltration, le circuit de liquide de dialyse est généralement soumis à des dépressions variables, ce qui peut entraîner des perturbations au niveau du fonctionnement de la pompe.

Les solutions proposées jusqu'ici dans l'art antérieur pour contrôler l'ultrafiltration du sang sont le contrôle du poids du patient au moyen d'une balance ou d'un lit balance, où la mesure du volume de liquide extrait par la pompe d'ultrafiltration au moyen d'un ou de plusieurs réservoirs calibrés, ainsi que cela est décrit dans la demande EP 213050.

De telles méthodes sont cependant insuffisantes car elles ne permettent que de constater à posteriori une dérive éventuelle de la pompe sans qu'il soit possible de corriger automatiquement son fonctionnement ; en outre, de telles méthodes ne peuvent pas être effectuées avec une fréquence suffisante pour permettre une grande exactitude.

Le brevet US 4 831 866 décrit un dispositif permettant de calibrer un débitmètre dans un distributeur de liquide.
Le distributeur comprend :
- une pompe permettant de pomper de l'essence à partir d'un réservoir,
- un débitmètre mécanique rotatif couplé à un émetteur d'impulsations,
- un dispositif d'affichage permettant d'effectuer la correspondance entre les impulsions émises et le volume fourni, grâce à une opération de multiplication entre le nombre d'impulsions émises et une constante d'affichage représentative de la relation entre le nombre d'impulsions reçues et le volume affiché.

Afin de s'affranchir des variations de fonctionnement du débitmètre, ce brevet propose un dispositif de calibration situé en dérivation entre la pompe et le débitmètre. Ce dispositif reçoit les impulsions provenant de l'émetteur d'impulsions et les corrige avant de les envoyer sur l'unité d'affichage.
Le dispositif de calibration comprend :
- un réservoir calibré muni d'un piston se déplaçant à l'intérieur de ce réservoir sous l'effet du liquide en mouvement,
- une vanne permettant de modifier le sens d'écoulement du liquide dans le réservoir,
- des détecteurs de niveau permettant de limiter précisément un volume connu à l'intérieur du réservoir et de déclencher le démarrage et la fin de la phase de comptage des impulsions,
- une unité centrale recevant des informations de l'émetteur d'impulsions ainsi que du réservoir calibré.

La correction des variations de fonctionnement du débitmètre est effectuée, par un comptage, lors de la phase de calibration des impulsions Pd émises par l'émetteur pendant la durée correspondant à la fourniture d'un volume calibré connu, par exemple le volume situé entre les 2 détecteurs de niveau. Le nombre Pd est comparé à un nombre prédéterminé ou obtenu précédemment, correspondant au nombre d'impulsions Nr devant être émises durant le pompage du volume calibré. Ce nombre Nr est utilisé pour établir un coefficient de calibration Nr/Pd par lequel est ensuite multiplié le nombre d'impulsions accumulées pendant la phase de fourniture du liquide.

Ce nombre d'impulsions corrigé est ensuite transmis à l'unité d'affichage qui, grâce à la constante d'affichage représentant la correspondance entre les impulsions émises et le volume fourni, peut afficher le volume réellement fourni.

Etant donné que ce dispositif de calibration est basé sur une correction du nombre d'impulsions émises par un émetteur, ce dispositif permet de constater a posteriori, avec une précision certes accrue par rapport aux pompes à essence de l'art antérieur, la quantité de liquide effectivement pompée.
Par contre, un tel dispositif ne permet pas d'agir directement sur la pompe afin d'imposer avec une grande précision la quantité et /ou le débit de liquide pompé.

Or, étant donné l'importance d'un contrôle très précis de l'ultrafiltration dans le traitement extracorporel du sang et les variations possibles dans le fonctionnement d'une pompe, il est essentiel de pallier les imperfections des méthodes et appareils de l'art antérieur.

L'objet de la présente invention est donc de proposer une méthode et un dispositif permettant de contrôler avec une grande précision le pompage de l'ultrafiltration du sang.

Un autre objet de la présente invention est de proposer une méthode et un dispositif permettant d'effectuer des contrôles du pompage de l'ultrafiltration du sang aussi fréquemment que souhaité.

Un autre objet de la présente invention est de proposer une méthode et un dispositif de contrôle du pompage de l'ultrafiltration du sang permettant de s'affranchir des conditions d'utilisation de la pompe.

Un autre objet de la présente invention est de proposer une méthode et un dispositif de contrôle du pompage de l'ultrafiltration permettant non seulement de vérifier la quantité réellement pompée mais également d'agir sur la pompe pour ajuster le pompage.
Afin d'atteindre ces différents buts, la présente invention propose un procédé de contrôle, dans un rein artificiel, de la quantité de liquide pompé dans un circuit, tel que défini dans la revendication 1.

La présente invention a également pour objet un dispositif de contrôle, dans un rein artificiel de la quantité de liquide pompé dans un circuit par une pompe , tel que défini dans la revendication 4.

D'autres objets et avantages apparaîtront au cours de la description qui va suivre, en référence aux dessins annexés qui illustrent, à titre d'exemples, et sans échelle déterminée, différents modes de réalisation de la présente invention.

La figure 1 représente, de façon schématique, un mode de réalisation simplifié de l'objet de la présente invention.

La figure 2 représente, de façon plus détaillée mais schématique, un mode de réalisation de la présente invention associé à différents éléments du contrôle de l'ultrafiltration.

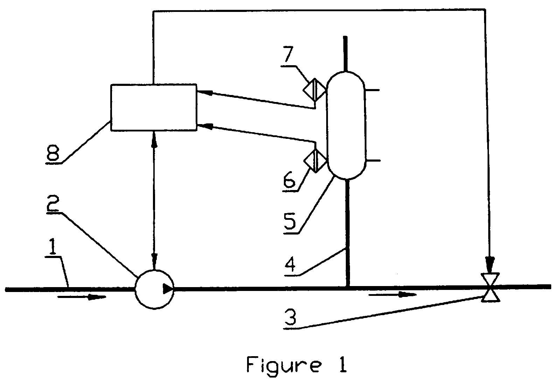

Selon le mode de réalisation représenté sur la figure 1, on dispose de façon classique d'une canalisation principale 1 dans laquelle circule du liquide mis en circulation par une pompe 2. Une vanne 3 est disposée sur la canalisation 1 en aval de la pompe 2. Selon l'invention, une canalisation de dérivation 4 de la canalisation principale 1 est située en aval de la pompe 2 et en amont de la vanne 3. Cette canalisation 4 conduit à un réservoir 5 muni d'un détecteur 6 de niveau bas et d'un détecteur 7 de niveau haut. La pompe 2, la vanne 3 ainsi que les détecteurs de niveau 6 et 7 sont reliés à un organe de contrôle 8. Le contrôle du fonctionnement de la pompe 2 est effectué de la manière décrite ci-après. La pompe 2 est une pompe occlusive telle qu'une pompe péristaltique, une pompe à diaphragme, à engrenage ou à palette, dont le fonctionnement se traduit par l'émission d'impulsions, chaque impulsion correspondant au passage d'une quantité déterminée de liquide. Les impulsions émises par la pompe réprésentent dans le cas d'une pompe péristaltique un certain angle de rotation du rotor alors que dans le cas d'une pompe à diaphragme, elles représentent un certain déplacement du diaphragme.
Ces impulsions sont transmises pour comptage à un organe de contrôle 8 qui est avantageusement un microprocesseur. Le microprocesseur 8 peut également effectuer des calculs concernant la quantité ou le débit de liquide pompé à partir du nombre d'impulsions enregistrées et de la valeur de la correspondance existant entre une impulsion émise et la quantité de liquide pompé en ml. Le but de l'opération de contrôle du fonctionnement de la pompe 2 est de réactualiser en permanence cette valeur de la quantité de liquide pompé par impulsion émise. Pour effectuer cette opération de contrôle, l'organe de contrôle 8 agit sur la vanne 3 afin de provoquer sa fermeture. Le liquide pompé par la pompe 2 s'écoule alors dans la canalisation de dérivation 4 pour remplir le réservoir 5. Le passage du liquide au niveau du détecteur 6 de niveau bas provoque un démarrage du comptage des impulsions par l'organe de contrôle 8. Le comptage se poursuit jusqu'à ce que le liquide parvienne au niveau du détecteur de niveau haut 7, ce qui déclenche au niveau de l'organe 8, d'une part un arrêt du comptage des impulsions et d'autre part, une action sur la vanne 3 pour provoquer son ouverture et permettre ainsi l'évacuation du liquide pompé vers l'égoût ou vers d'autres moyens d'élimination.

La capacité Vo du réservoir 5 entre les deux détecteurs 6 et 7 est parfaitement connue et constitue une des données mémorisées par l'organe 8 qui peut, à partir du nombre d'impulsions No comptées pendant le temps de remplissage du réservoir 5, calculer la valeur Vo/No de la quantité de liquide réellement pompée par impulsion émise.

Naturellement, il est tout à fait possible qu'au lieu d'effectuer le rapport Vo/No, l'organe 8 contrôle le fonctionnement de la pompe 2 en calculant le rapport No/Vo, c'est-à-dire le nombre d'impulsions émises pour une certaine quantité de liquide pompé, par exemple, par ml de liquide pompé.

Ainsi, selon l'invention, on réactualise en permanence la correspondance existant entre les impulsions émises par la pompe et la quantité de liquide pompé, on détecte et on corrige ainsi les variations pouvant se produire dans le fonctionnement de la pompe 2 selon ses conditions d'utilisation. Cela permet de s'affranchir des variations de performance pouvant survenir en fonction de l'usure de la pompe ou de la pression exercée en amont de la pompe.

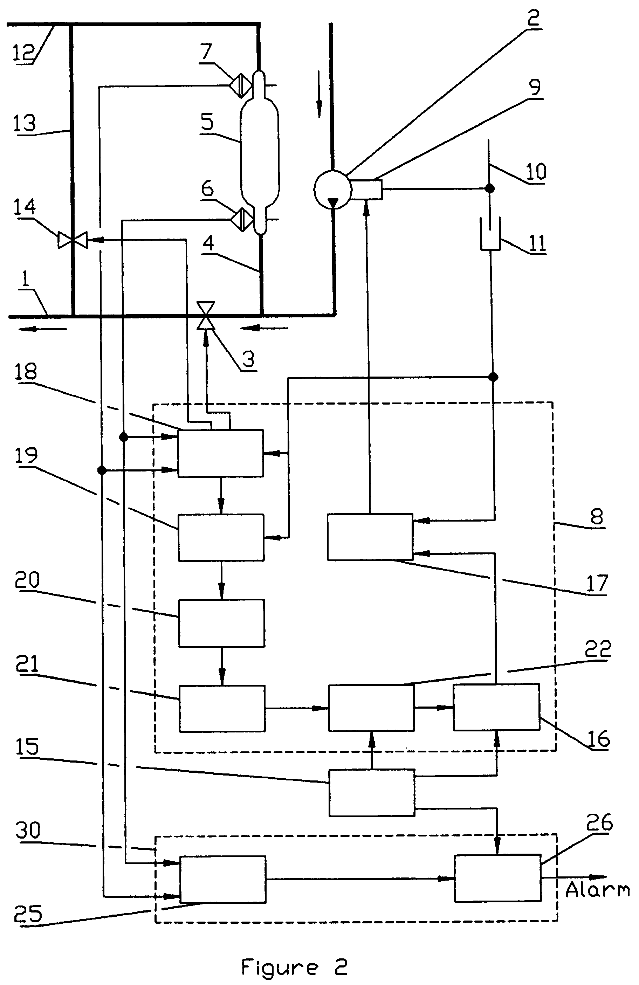

La figure 2 représente, de façon plus détaillée, un mode de réalisation de la présente invention, intégré au fonctionnement de la pompe d'ultrafiltration 2. De façon classique, cette pompe 2 est mise en marche grâce à un moteur 9 dont la rotation est contrôlée par un système 10 émettant des impulsions correspondant à un certain angle de rotation du moteur. Un tel système est constitué, par exemple, par une roue dentée dont l'axe de rotation est le même que celui du moteur 9. Un détecteur optique 11 contrôle la rotation de la roue par détection d'un faisceau lumineux qui est arrêté par les zones correspondant aux dents de la roue et qui est transmis par les parties vides situées entre deux dents.

La pompe 2 est alimentée par du liquide de dialyse provenant du circuit de liquide de dialyse (non représenté) de préférence en aval de l'échangeur. Le liquide pompé s'écoule dans la canalisation 1 dans le sens symbolisé par la flèche. Cette canalisation 1 est munie d'une vanne 3, en amont de laquelle est située une dérivation 4 conduisant à un réservoir 5. Ce réservoir est pourvu de détecteurs de niveau 6 et 7 ; il est relié par une canalisation 12 à une source d'air (non représentée) permettant d'assurer sa vidange. Cette canalisation 12 est reliée en aval de la vanne 3 à la canalisation 1 conduisant le liquide pompé à des moyens d'élimination (non représentés) par une canalisation 13 munie d'une vanne 14. Les vannes 3 et 14 sont commandées par l'organe de contrôle 8.

Les différents éléments de l'organe de contrôle 8 qui sont nécessaires au fonctionnement de l'objet de l'invention sont représentés de façon schématique par des rectangles numérotés ; les fonctions qu'ils assurent sont explicitées dans la description ci-dessous.

L'élément 15 assure l'interface entre l'organe de contrôle 8 et l'utilisateur ; il peut être constitué par un écran et un clavier, par un bouton ou par tout autre moyen permettant d'introduire des données dans l'organe 8 constitué avantageusement par un microprocesseur.

L'interface 15 permet à l'utilisateur d'introduire dans le microprocesseur 8 la valeur en débit ou en quantité (en volume ou en poids) de la perte de poids désirée du patient. Dans le cas où le patient n'absorbe aucun élément pendant la séance et où on ne réinjecte aucun liquide dans le sang qui doit lui être restitué, cette perte de poids désirée correspond directement à l'ultrafiltration du sang que l'on souhaite effectuer. Dans le cas où, à l'inverse, on réinjecte au patient une certaine quantité de liquide tel que cela se fait pour l'hémodiafiltration, ou bien dans le cas où le patient mange et/ou boit pendant la séance de traitement, la valeur de ces apports est introduite dans le microprocesseur 8 qui calcule alors l'ultrafiltration du sang qui est nécessaire afin d'atteindre, en fin de séance, la perte de poids désirée. Il est également possible de calculer l'ultrafiltration à effectuer en dehors du microprocesseur et d'entrer directement cette valeur de l'ultrafiltration à réaliser au moyen de l'interface 15. Dans certains cas, tels que dans le contrôle volumétrique de l'ultrafiltration, fixer la valeur de l'ultrafiltration revient à fixer la quantité ou le débit de liquide que la pompe 2 doit extraire du circuit de liquide de dialyse. Cette donnée est entrée dans un registre 16 du microprocesseur, qui peut être considéré comme une mémoire et qui, à partir des caractéristiques connues de la pompe ou des caractéristiques précédemment vérifiées, peut transformer cette exigence d'ultrafiltration en consigne de fréquence de rotation du moteur 9 de la pompe 2. Cette consigne est transmise à l'organe d'asservissement 17 qui reçoit également des informations provenant du détecteur 11 relatives à la fréquence de rotation de la roue dentée 10 et traduisant donc la fréquence de rotation du moteur 9. On a ainsi une boucle d'asservissement du moteur de la pompe 2.

Selon l'invention, la correspondance existant entre les quantités de liquide délivrées par la pompe 2 et les impulsions émises, est contrôlée grâce à l'élément d'étalonnage 18 du microprocesseur. Afin d'effectuer l'étalonnage de la pompe 2, l'élément 18 commande la fermeture de la vanne 3 et l'ouverture de la vanne 14, ce qui permet à l'air se trouvant dans la canalisation 12 d'être évacué par la canalisation 1 vers des moyens d'élimination (non représentés) tels que l'égoût. Le liquide de dialyse pompé par la pompe 2 s'écoule alors dans la canalisation de dérivation 4 et dans le réservoir 5. Lorsque le liquide passe devant le détecteur de niveau bas 6, celui-ci transmet un signal à l'organe d'étalonnage 18 qui commence alors à compter les impulsions provenant du détecteur 11. Le comptage se poursuit jusqu'à ce que le liquide atteigne le niveau haut marqué par le détecteur 7 qui émet alors un signal à l'organe d'étalonnage 18 qui arrête aussitôt le comptage, commande l'ouverture de la vanne 3 et la fermeture de la vanne 14, provoque la vidange du réservoir 5. L'air nécessaire à la vidange du réservoir 5 peut provenir de n'importe quelle source appropriée. Cependant, de façon particulièrement avantageuse, on utilise dans la présente invention, l'air provenant du dégazage du liquide de dialyse effectué en amont de l'échangeur. La capacité Vo du réservoir 5 entre les deux détecteurs 6 et 7 étant parfaitement fixée et connue de l'organe 18, celui-ci peut, à partir du nombre No d'impulsions enregistrées pendant le temps de comptage, calculer un coefficient d'étalonnage K = Vo/No qui correspond à la quantité de liquide pompé par impulsion émise. A chaque opération d'étalonnage, s'effectuant tel que cela vient d'être décrit, la valeur du coefficient de calibration est ainsi réactualisée.

La valeur de ce coefficient K est transmise au multiplicateur 19 qui reçoit également des informations concernant le nombre d'impulsions émises par la roue. Chaque impulsion est multipliée par le coefficient d'étalonnage K précédemment calculé, ce qui donne une valeur de la quantité de liquide pompé qui est transmise à un totalisateur 20 qui ajoute cette quantité de liquide pompé à la quantité précédemment enregistrée. Ainsi, ce totalisateur 20 est incrémenté à chaque impulsion ou à chaque groupe d'impulsions ; on peut en effet choisir de ne pas effectuer la multiplication des impulsions par le coefficient d'étalonnage, pour chaque impulsion émise, mais seulement pour chaque groupe d'impulsions, par exemple toutes les 50, 100 ou 1000 impulsions.

La nouvelle valeur ainsi obtenue de la quantité de liquide pompé, c'est-à-dire de l'ultrafiltration réalisée, peut alors être affichée, soit directement, soit après conversion en perte de poids tenant compte des différents apports faits au patient sous forme d'aliments ou d'injection, ce qui permet à la fois au patient et au personnel médical de s'assurer que la séance se déroule comme souhaité.

La valeur de l'ultrafiltration est transmise à un diviseur 21 qui, grâce à une mesure du temps écoulé, calcule le débit d'ultrafiltration. Cette valeur du débit d'ultrafiltration réalisé est comparée par l'élément 22 à la valeur provenant de l'interface 15 du débit souhaité par l'utilisateur. Dans le cas où la différence entre les deux débits se situe en dehors d'une fourchette fixée, l'élément 22 réactualise le registre 16 pour ajuster la consigne de fréquence de rotation du moteur de la pompe au débit désiré. Ainsi, selon l'invention, les variations pouvant survenir dans le fonctionnement de la pompe 2 sont non seulement constatées mais également corrigées et la valeur d'ultrafiltration affichée est la valeur de l'ultrafiltration effectivement réalisée. En outre, la réactualisation de la consigne de fréquence du moteur de la pompe au débit d'ultrafiltration désiré permet d'imposer avec un grande précision l'ultrafiltration à effectuer et de ne pas se contenter simplement de constater à postériori l'ultrafiltration réalisée.

L'étalonnage peut être effectué, à la demande de l'utilisateur, ou de façon permanente dès qu'un cycle de remplissage et de vidange est effectué, avec cependant une temporisation de sécurité pour que l'on soit bien certain d'avoir complètement vidé le réservoir 5. Dans le cas où on choisit un réservoir de 10 ml, si la pompe 2 agit de manière à effectuer une ultrafiltration de 100 ml/heure, le temps de remplissage du réservoir sera de 6 minutes. Le temps de vidange étant, selon l'air disponible, de l'ordre de 2 minutes, l'étalonnage sera donc effectué environ toutes les 8 à 10 minutes. Si par contre, l'ultrafiltration désirée est de 1 l/heure, ce qui représente une valeur commune, le temps de remplissage du réservoir ne sera plus que de 36 secondes. On effectuera alors un étalonnage toutes les 3 minutes environ.

De nombreuses variantes dans l'utilisation de la réactualisation du coefficient d'étalonnage sont à la portée du technicien, sans pour autant sortir du cadre de la présente invention.

Il est également possible d'utiliser les données provenant du dispositif d'étalonnage pour effectuer certains contrôles de sécurité. En effet, les données provenant des détecteurs de niveau 6 et 7 peuvent être transmises à un microprocesseur 30 et plus particulièrement à un élément 25 de ce microprocesseur qui mesure le temps nécessaire au remplissage du réservoir entre les niveaux 6 et 7. Cet élément possède en mémoire la valeur Vo du volume du réservoir entre les deux détecteurs et peut ainsi calculer le débit du pompage de liquide Vo/t. Cette valeur du débit est transmise à un comparateur 26 qui compare cette valeur à celle provenant de l'interface 15 qui représente le débit souhaité par l'utilisateur. Dans le cas où la différence entre les deux valeurs est supérieure à une limite prédéterminée, le microprocesseur 30 déclenche une alarme, permettant d'alerter le personnel médical chargé de la surveillance de la séance de traitement.

Le dispositif et la méthode de contrôle du pompage de liquide par la pompe 2 permet, ainsi que cela a été décrit plus haut, d'une part de vérifier avec une grande exactitude la quantité de liquide réellement pompé, et d'autre part, grâce à la réactualisation de la consigne de fréquence du moteur en fonction du débit souhaité, d'agir sur la pompe 2 afin que la quantité de liquide réellement pompée soit exactement la quantité souhaitée.

Cette possibilité d'imposer, et non pas seulement de constater, la quantité de liquide pompée, présente un intérêt particulier dans le cas où le liquide pompé est extrait du circuit de liquide de dialyse et correspond à la quantité de liquide extrait du sang par ultrafiltration.

## Revendications

1. Procédé de contrôle, dans un rein artificiel, de la quantité de liquide pompé dans un circuit, caractérisé en ce qu'il consiste à :
- pomper le liquide grâce à une pompe (2) ayant un rotor ou un diaphragme, associée à des moyens (10) émettant des impulsions correspondant chacune au passage d'une certaine quantité de liquide et représentant un certain angle de rotation du rotor ou un certain deplacement du diaphragme,
- compter le nombre d'impulsions émises,
- déterminer la quantité de liquide pompé à partir du nombre d'impulsions émises et la correspondance existant entre le nombre d'impulsions et la quantité ce liquide pompé,
- à étalonner la pompe automatiquement et sans arrêter son fonctionnement, en :
- faisant circuler le liquide pompé dans un réservoir (5) de capacité Vo connue avec exactitude,
- comptant le nombre d'impulsions No émises pour remplir ledit réservoir (5),
- établissant la correspondance exacte existant entre le nombre d'impulsions No émises et la quantité Vo de liquide pompé.

2. Procédé selon la revendication 1, caractérisé en ce qu'il consiste en outre à :
- déterminer le débit réel de pompage du liquide,
- comparer le débit réel avec le débit désiré,
- fixer des limites d'admissibilité au produit de comparaison du débit réel avec le débit désiré,
- corriger la vitesse de fonctionnement de la pompe dans le cas où le produit de la comparaison entre le débit réel et le débit désiré se situe en dehors des limites fixées.

3. Procédé selon une des revendications précédentes, caractérisé en ce que le liquide pompé est du liquide de dialyse pompé en quantité égale à la quantité de liquide que l'on souhaite extraire du sang par ultrafiltration.

4. Dispositif de contrôle, dans un rein artificiel, de la quantité de liquide pompé dans un circuit par une pompe ayant un rotor ou un diaphragme (2): caractérisé en ce qu'il comporte:
- des moyens (10) pour émettre des impulsions correspondant chacune au passage d'une certaine quantité de liquide et représentant un certain angle de rotation du rotor ou un certain deplacement du diaphragme: - des moyens (18) pour compter les impulsions émises, - des moyens pour déterminer la quantité de liquide pompé à partir du nombre d'impulsions émises et la correspondance existant entre le nombre d'impulsions et la quantité de liquide pompé,
- des moyens pour étalonner automatiquement la pompe (2) sans arrêter son fonctionnement, lesdits moyens comprenant notamment :
- des moyens (5,6,7) pour mesurer un volume Vo exactement déterminé du liquide pompé,
- des moyens (18) pour compter le nombre d'impulsions No émises pendant le pompage du volume Vo,
- des moyens (18) pour établir la correspondance exacte existant entre le nombre d'impulsions émises No et la quantité Vo de liquide pompé.

5. Dispositif selon la revendication 4, caractérisé en ce qu'il comporte en outre :
- des moyens (21) pour déterminer le débit réel du liquide pompé,
- des moyens (22) pour comparer le débit réel avec le débit désiré,
- des moyens (16) pour corriger la vitesse de fonctionnement de la pompe en fonction du produit de la comparaison entre le débit réel et le débit désiré.

6. Dispositif selon une des revendications 4 ou 5, caractérisé en ce que lesdits moyens (5,6,7) pour mesurer un volume Vo exactement déterminé du liquide pompé sont situés en aval des moyens de pompage (2) par rapport au sens d'écoulement du liquide pompé.

7. Dispositif selon une des revendications 4 à 6, caractérisé en ce que le liquide pompé est du liquide de dialyse pompé en quantité égale à la quantité de liquide que l'on souhaite extraire du sang par ultrafiltration.

## Claims

1. Method for controlling, in an artificial kidney, the quantity of liquid pumped in a circuit, characterized in that it consists in :
- pumping the liquid by means of a pump (2) having a rotor or a diaphragm, the pump being associated with means (10) emitting impulses which each corresponds to the passage of a certain quantity of liquid, and represents a certain angle of rotation of the rotor or a certain displacement of the diaphragm,
- counting the number of impulses emitted,
- determining the quantity of liquid pumped on the basis of the number of impulses emitted and the correspondence existing between the number of impulses and the quantity of liquid pumped,
- calibrating the pump automatically and without stopping its operation, by:
- circulating the liquid pumped in a reservoir (5) which is of an exactly known capacity Vo,
- counting the number of impulses No emitted in order to fill the said reservoir (5),
- establishing the exact correspondence existing between the number of impulses No emitted and the quantity Vo of liquid pumped.

2. Method according to Claim 1, characterized in that it additionally consists in:
- determining the actual flowrate of pumping of the liquid,
- comparing the actual flowrate with the desired flowrate,
- fixing admissible limits in respect of the result of the comparison of the actual flowrate with the desired flowrate,
- correcting the operational speed of the pump in the case where the result of the comparison between the actual flowrate and the desired flowrate is situated outside the fixed limits.

3. Method according to one of the preceding claims, characterized in that the liquid pumped is dialysis liquid pumped in a quantity equal to the quantity of liquid which it is desired to extract from the blood by ultrafiltration.

4. Device for controlling, in an artificial kidney, the quantity of liquid pumped in a circuit by a pump (2) having a rotor or a diaphragm, characterized in that it comprises :
- means (10) for emitting impulses which each corresponds to the passage of a certain quantity of liquid, and represents a certain angle of rotation of the rotor or a certain displacement of the diaphragm,
- means (18) for counting the impulses emitted,
- means for determining the quantity of liquid pumped on the basis of the number of impulses emitted and the correspondence existing between the number of impulses and the quantity of liquid pumped,
- means for automatically calibrating the pump (2) without stopping its operation, the said means comprising in particular:
- means (5, 6, 7) for measuring an exactly determined volume Vo of the liquid pumped,
- means (18) for counting the number of impulses No emitted during the pumping of the volume Vo,
- means (18) for establishing the exact correspondence existing between the number of impulses emitted No and the quantity Vo of liquid pumped.

5. Device according to Claim 4, characterized in that it additionally comprises:
- means (21) for determining the actual flowrate of the liquid pumped,
- means (22) for comparing the actual flowrate with the desired flowrate,
- means (16) for correcting the operational speed of the pump as a function of the result of the comparison between the actual flowrate and the desired flowrate.

6. Device according to either of Claims 4 or 5, characterized in that the said means (5, 6, 7) for measuring an exactly determined volume Vo of the liquid pumped are situated downstream of the pumping means (2) with respect to the direction of flow of the liquid pumped.

7. Device according to one of Claims 4 to 6, characterized in that the liquid pumped is dialysis liquid pumped in a quantity equal to the quantity of liquid which it is desired to extract from the blood by ultrafiltration.

## Patentansprüche

1. Verfahren zur Kontrolle der in einem Kreislauf gepumpten Flüssigkeitsmenge in einer künstlichen Niere, dadurch gekennzeichnet, daß es folgendes umfaßt :
- Pumpen der Flüssigkeit mit einer Pumpe (2), die mit einem Rotor oder einer Membran versehen ist und die mit Mitteln (10) verbunden ist, welche Impulse emittieren, von denen jeder dem Durchfluß einer gewissen Flüssigkeitsmenge entspricht, und einen gewissen Rotationswinkel des Rotors oder eine gewisse Auslenkung der Membran darstellt,
- Zählen der Anzahl emittierter Impulse,
- Bestimmen der gepumpten Flüssigkeitsmenge auf der Grundlage der Anzahl emittierter Impulse und der zwischen der Impulsanzahl und der gepumpten Flüssigkeitsmenge bestehenden Beziehung,
- automatisches Eichen der Pumpe, und ohne ihren Betrieb anzuhalten, durch :
- Zirkulierenlassen der gepumpten Flüssigkeit in dem Behälter (5) der genau bekannten Kapazität Vo,
- Zählen der Anzahl der emittierten Impulse No, um den Behälter (5) zu füllen,
- Einrichten der genauen, zwischen der Anzahl der emittierten Impulse No und der Menge der gepumpten Flüssigkeit Vo bestehenden Beziehung.

2. Verfahren gemäß Anspruch 1, dadurch gekennzeichnet, daß es zudem umfaßt:
- Bestimmen der tatsächlichen Rate der Flüssigkeitspumparbeit,
- Vergleichen der tatsächlichen Rate mit dem gewünschten Rate,
- Festlegen der Zulässigkeitsgrenzen des Vergleichsergebnisses der tatsächlichen Rate mit der gewünschten Rate,
- Korrigieren der Betriebsgeschwindigkeit der Pumpe in dem Fall, in dem das Vergleichsergebnis zwischen der tatsächlichen Rate und der gewünschten Rate außerhalb der festgelegten Grenzen liegt.

3. Verfahren gemäß einem der vorhergehenden Patentansprüche, dadurch gekennzeichnet, daß die gepumpte Flüssigkeit eine Dialyseflüssigkeit ist, die in gleicher Menge wie die Flüssigkeitsmenge, die mit der Ultrafiltration aus dem Blut extrahiert werden soll, gepumpt wird.

4. Vorrichtung zur Kontrolle der Menge der Flüssigkeit in einem Kreislauf einer künstlichen Niere, wobei die Flüssigkeit mit einer Pumpe (2) gepumpt ist, die mit einem Rotor oder einer Membran versehen ist, dadurch gekennzeichnet, daß sie folgendes umfaßt :
- Mittel (10) zum Emittieren von Impulsen, von denen jeder dem Durchfluß einer gewissen Flüssigkeitsmenge entspricht, und einen gewissen Rotationswinkel des Rotors oder eine gewisse Auslenkung der Membran darstellt,
- Mittel (18) zum Zählen der emittierten Impulse,
- Mittel zum Bestimmen der Menge der gepumpten Flüssigkeit auf der Grundlage der Anzahl der emittierten Impulse und der zwischen der Impulsanzahl und der gepumpten Flüssigkeitsmenge bestehenden Beziehung,
- Mittel zum automatischen Eichen der Pumpe (2) ohne den Betrieb der Pumpe anzuhalten, wobei die Mittel insbesondere umfassen:
- Mittel (5, 6, 7) zum Messen eines genau bestimmten Volumens Vo der gepumpten Flüssigkeit,
- Mittel (18) zum Zählen der Anzahl No der während des Pumpens des Volumens Vo emittierten Impulse,
- Mittel (18) zum Einrichten der genauen zwischen der Anzahl der emittierten Impulse No und der Menge Vo der gepumpten Flüssigkeitsmenge bestehenden Beziehung.

5. Vorrichtung gemäß Anspruch 4, dadurch gekennzeichnet, daß sie zusätzlich umfaßt:
- Mittel (21) zum Bestimmen der tatsächlichen Rate der gepumpten Flüssigkeit,
- Mittel (22) zum Vergleichen der tatsächlichen Rate mit der gewünschten Rate,
- Mittel (16) zum Korrigieren der Arbeitsgeschwindigkeit der Pumpe im Zusammenhang mit dem Vergleichsergebnis zwischen der tatsächlichen Rate und der gewünschten Rate.

6. Vorrichtung gemäß einem der Ansprüche 4 oder 5, dadurch gekennzeichnet, daß die Mittel (5, 6, 7) zum Messen eines genau bestimmten Volumens Vo der gepumpten Flüssigkeit stromabwärts der Mittel zum Pumpen (2) in bezug auf die Fließrichtung der gepumpten Flüssigkeit angeordnet sind.

7. Vorrichtung gemäß einem der Ansprüche 4 bis 6, dadurch gekennzeichnet, daß die gepumpte Flüssigkeit eine Dialyseflüssigkeit ist, die in gleicher Menge wie die Menge der Flüssigkeit gepumpt wird, die mit der Ultrafiltration aus dem Blut extrahiert werden soll.
